# EUROPEAN PATENT APPLICATION

(11) **EP 3 628 660 A1**
(43) Date of publication of application: **01.04.2020**
(21) Application number: 18306280.1
(22) Date of filing: 28.09.2018
(51) Int. Cl.: C07D 211/32, A61K 31/445, A61P 25/28

(54) **DONECOPRIDE AND FLUCOPRIDE AS NEUROPROTECTIVE AGENTS IN THE TREATMENT OF NEURODEGENERATIVE DISEASES**

(71) Applicant: Université de Caen Normandie, 14000 Caen (FR); INSTITUT NATIONAL DE LA SANTE ET DE LA RECHERCHE MEDICALE (INSERM), 75013 Paris (FR); CENTRE NATIONAL DE LA RECHERCHE SCIENTIFIQUE, 75016 Paris (FR); Université de Montpellier, 34090 Montpellier (FR)
(72) Inventor: DALLEMAGNE, Patrick, 14260 Seulline (FR); ROCHAIS, Christophe, 14000 Caen (FR); CLAEYSEN, Sylvie, 34980 Saint-Gely-du-Fesc (FR)
(74) Representative: Lavoix

(57) **Abstract**

The present invention concerns a compound of formula (I) for use as neuroprotective agent in the prevention and/or treatment of a neurodegenerative disease.

## Description

The present invention concerns the treatment of neurodegenerative diseases.

Neurodegenerative diseases are those which are induced by the collapse of nervous circuit neural network based on the systematic degeneration and deciduation of neurocytes, and they have been known as various intractable diseases such as Alzheimer's disease (AD), Parkinson's disease, Lewis body dementia, vascular dementia, and problem associated with ageing including (i) non-cognitive neurodegeneration, (ii) non-cognitive neuromuscular degeneration and (iii) motor-sensory neurodegeneration, Huntington's disease, motor neurone diseases including amyotrophic lateral sclerosis (ALS), Down syndrome, corticobasal ganglionic degeneration, multiple system atrophy, cerebral atrophy, olivopontocerebellar atrophy, supranuclear palsy, Friedreich's ataxia, spinocerebellar ataxia type 2, frontotemporal degenerations (FTD) and primary progressive aphasia.

Clinical symptoms of neurodegenerative diseases vary from minor ones to severe ones, depending on the disease. They include tremor, rigidity, akinesia, hypokinesia, bradykinesia, attitude reflex failure, dysautonomia, pulsion, gait disturbance, depression, apathy, confusion, learning alteration, memory deficits, cognitive impairment, amyotrophia, muscle loss, disorder of shoulder girdle, articulation disorder, dysphagia, respiration disorder, numbness, paralysis and dementia, which are major hurdles in performing daily activities.

Neurodegenerative death is a complicated mechanism probably due to multiple molecular groups which trigger neurodegenerative diseases when issues in their expression, activity and/or regulation occur. This may explain why no effective method for inhibiting or reversing neurodegeneration has been established so far.

In addition to the treatment for removing causatives of such diseases, it is also important to reconstruct the neural network. For example, there have been said that, in Alzheimer's disease for which cytotoxicity of amyloid β peptide has been recognized as a causative, both the atrophia of neurites and the reduction of synapses trigger off the deterioration of neurofunction, and in reverse, even after such triggering, neurocytes which are not fully denatured or survived without degeneration can be recovered if they can possibly be activated to extend neurites for recovering synapse.

There is thus an important need for new agents capable of protecting neurites but also able to increase neurite growth and to promote the formation of new synapses, in order both to prevent and curatively treat neurodegenerative diseases.

The present invention meets this need.

The present invention indeed results from the unexpected finding by the inventors that the Donecopride compound, disclosed in the international application WO 2014195593, has a neuroprotective activity since it is able, in addition to decrease Tau hyperphosphorylation, to increase neurite growth, to maintain the synapses rate, and most importantly and unexpectedly to promote formation of new synapses.

Because of this general and efficient protective effect on neurites, the Donecopride compound is a very promising neuroprotective agent for preventing and/or treating any neurodegenerative disease involving neurites degeneration.

The present invention thus concerns a compound of the following formula (I): 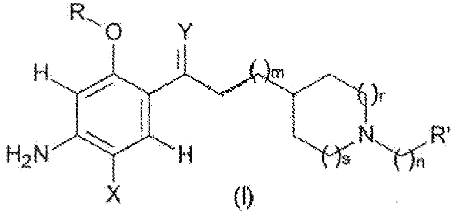 wherein:
X represents
   a hydrogen atom, or
   a halogen atom (Hal), where (Hal) is fluorine, chlorine, bromine or iodine, or
   a straight- or branched-chain Cₚ(Hal)₂ₚ₊₁ polyhalogenoalkyl group, where p=1, 2, 3 or 4, (Hal) having the same meaning as indicated above;
Y represents
   an oxygen atom, or
   a sulfur atom, or
   an N-R" radical where R" represents a hydrogen atom, an -OH radical or a straight- or branched-chain C_{q}H_{2q+1} alkyl radical, where q=1, 2, 3 or 4;
m is an integer selected from 1, 2 and 3;
n is an integer selected from 0, 1, 2 and 3;
r and s are integers whose values are: r=s=0; or r=s=1; or r=s=2; or r=0 and s=1; or lastly r=0 and s=2;
R represents
   a hydrogen atom, or
   a straight- or branched-chain C₁-C₅ alkyl group capable of carrying one or more F atoms;
R' represents
   a branched-chain C₁-C₆ alkyl radical, or
   a C₃-C₁₀ cycloalkyl or C₅-C₁₃ bicyclic group, capable of carrying one or more R groups and of possessing an oxygen atom, or a nitrogen atom that can be substituted by R, or a sulfur atom or a radical -SO₂₋ or -SO-,
as well as its enantiomers or diastereoisomers and its racemics, its acid salts, its hydrates or its solvation products,
for use as neuroprotective agent in the prevention and/or treatment of a neurodegenerative disease.

### Detailed description of the invention

### Compounds

The compound used in the context of the invention is a compound of the following formula (I): 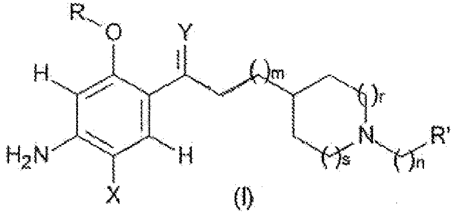 wherein:
X represents
   a hydrogen atom, or
   a halogen atom (Hal), where (Hal) is fluorine, chlorine, bromine or iodine, or
   a straight- or branched-chain Cₚ(Hal)₂ₚ₊₁ polyhalogenoalkyl group, where p=1, 2, 3 or 4, (Hal) having the same meaning as indicated above;
Y represents
   an oxygen atom, or
   a sulfur atom, or
   an N-R" radical where R" represents a hydrogen atom, an -OH radical or a straight- or branched-chain C_{q}H_{2q+1} alkyl radical, where q=1, 2, 3 or 4;
m is an integer selected from 1, 2 and 3;
n is an integer selected from 0, 1, 2 and 3;
r and s are integers whose values are: r=s=0; or r=s=1; or r=s=2; or r=0 and s=1; or lastly r=0 and s=2;
R represents
   a hydrogen atom, or
   a straight- or branched-chain C₁-C₅ alkyl group capable of carrying one or more F atoms;
R' represents
   a branched-chain C₁-C₆ alkyl radical, or
   a C₃-C₁₀ cycloalkyl or C₅-C₁₃ bicyclic group, capable of carrying one or more R groups and of possessing an oxygen atom, or a nitrogen atom that can be substituted by R, or a sulfur atom or a radical -SO₂₋ or -SO-,
as well as its enantiomers or diastereoisomers and its racemics, its acid salts, its hydrates or its solvation products.

In a particular embodiment, in the formula (I), X represents a halogen atom, in particular a chlorine or a fluorine, more particularly a chlorine.

In another particular embodiment, in the formula (I), Y represents an oxygen atom.

In another particular embodiment, in the formula (I), all of m, n, r and s have the value 1.

In another particular embodiment, in the formula (I), R represents H, CH₃, CH₂CH₃ or CH₂-CH₂F. Preferably, R represents CH₃.

In another particular embodiment, in the formula (I), R' represents a radical selected from the group consisting in the radicals cyclopropyl, cyclobutyl, cyclopentyl, cyclohexyl, 4-piperidine and -CH₂NHSO₂CH₃. Preferably, in the formula (I), R' represents a radical selected from the group consisting in the radicals cyclopropyl, cyclobutyl, cyclopentyl, cyclohexyl, 4-piperidine. Still preferably, R' represents a C₄-C₇ cycloalkyl radical. Most preferably, R' represents a cyclohexyl radical.

In another particular embodiment, in the formula (I), R represents CH₃ and R' represents a C₄-C₇ cycloalkyl radical, in particular a cyclohexyl radical.

In a particularly preferred embodiment, in the formula (I), X represents a chlorine, Y represents an oxygen atom, all of m, n, r and s have the value 1, R represents CH₃ and R' represents a cyclohexyl radical. In other words, in a particularly preferred embodiment, the compound used in the context of the invention is the donecopride compound of following formula (II):

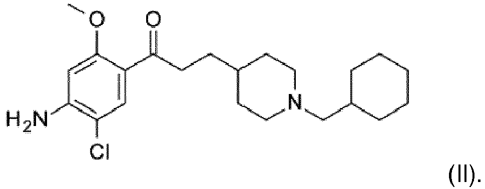

In another particularly preferred embodiment, in the formula (I), X represents a fluorine, Y represents an oxygen atom, all of m, n, r and s have the value 1, R represents CH₃ and R' represents a cyclohexyl radical. In other words, in a particularly preferred embodiment, the compound used in the context of the invention is the flucopride compound of following formula (III):

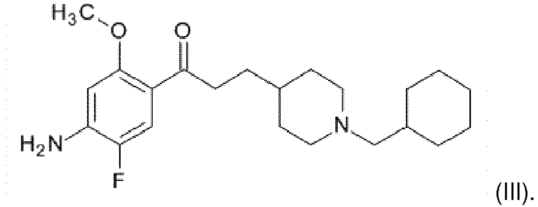

### Neurodegenerative disease

By "neurodegenerative disease" is meant herein a disease induced by the collapse of nervous circuit neural network based on the systematic degeneration and deciduation of neurocytes.

As explained above, the present inventors demonstrated that Donecopride displayed a general and efficient protective effect on neurites. Because of this general neuroprotective effect, Donecopride can be used as neuroprotective agent for preventing and/or treating any neurodegenerative disease.

Examples of neurodegenerative disease include Alzheimer's disease (AD), Parkinson's disease, Lewis body dementia, vascular dementia, and problem associated with ageing including (i) non-cognitive neurodegeneration, (ii) non-cognitive neuromuscular degeneration and (iii) motor-sensory neurodegeneration, Huntington's disease, motor neurone diseases including amyotrophic lateral sclerosis (ALS), Down syndrome, corticobasal ganglionic degeneration, multiple system atrophy, cerebral atrophy, olivopontocerebellar atrophy, supranuclear palsy, Friedreich's ataxia, spinocerebellar ataxia type 2, frontotemporal degenerations (FTD) and primary progressive aphasia.

In a particular embodiment, the neurodegenerative disease is selected from the group consisting of Alzheimer's disease, Parkinson's disease, Huntington's disease, multiple system atrophy, motor neurone diseases including amyotrophic lateral sclerosis, Down syndrome and frontotemporal degenerations.

In another embodiment, the neurodegenerative disease is Alzheimer's disease.

In an alternative embodiment, the neurodegenerative disease is a neurodegenerative disease which is not Alzheimer's disease.

### Medical indications

The present invention relates to a compound as defined in the section *"Compounds"* above for use as neuroprotective agent in the prevention and/or treatment of a neurodegenerative disease as defined in the section *"Neurodegenerative disease"* above.

The present invention also concerns the use of a compound as defined in the section *"Compounds"* above for the manufacture of a neuroprotective agent for the prevention and/or treatment of a neurodegenerative disease as defined in the section *"Neurodegenerative disease"* above.

The present invention further concerns a method of preventing and/or treating a neurodegenerative disease as defined in the section *"Neurodegenerative disease"* above, comprising administering to a subject in need thereof a therapeutically efficient amount of a compound as defined in the section *"Compounds"* above as neuroprotective agent.

The present invention further concerns a method of neuroprotection in a subject suffering from, or at risk of suffering from a neurodegenerative disease as defined in the section *"Neurodegenerative disease"* above, comprising administering to said subject a therapeutically efficient amount of a compound as defined in the section *"Compounds"* above.

According to the invention, the term "subject" or "subject in need thereof" is intended for a human or non-human mammal affected or likely to be affected with a neurodegenerative disease as defined in the section *"Neurodegenerative diseases"* above.

In the context of the invention, the term "treating" or "treatment" means reversing, alleviating or inhibiting the progress of the disorder or condition to which such term applies, or one or more symptoms of such disorder or condition.

In the context of the invention, the term "preventing" or "prevention" means delaying or preventing the onset of the disorder or condition to which such term applies, or one or more symptoms of such disorder or condition.

By "neuroprotection" is meant herein the prevention or inhibition of degenerative effects and restauration of neuronal integrity in the central nervous system, in particular the prevention or inhibition of neuronal degeneration.

By "neuroprotective agent" is meant herein an agent able of neuroprotection as defined above.

The inventors more particularly demonstrated that the compounds used in the context of the invention were capable of increasing neurite growth, maintaining synapses rate, decreasing Tau hyperphosphorylation and/or promoting formation of new synapses.

Accordingly, in a particular embodiment, the compound as defined in the section *"Compounds"* above is for increasing neurite growth.

The present invention also concerns a method for increasing neurite growth in a subject suffering from, or at risk of suffering from a neurodegenerative disease as defined in the section *"Neurodegenerative disease"* above, comprising administering to said subject a therapeutically efficient amount of a compound as defined in the section *"Compounds"* above.

By "neurite growth" is meant herein the extension process of any projection from the cell body of a neuron and associated synapse genesis.

By "increasing neurite growth" is meant herein that the growth of neurites is higher (*i.e.* more efficient, faster or longer), preferably statistically significantly higher, when the compound of interest is applied compared to the growth of neurites observed in the same conditions but without applying the compound of interest. Preferably, the neurite growth is increased of at least 120% compared to growth of neurites observed in the same conditions but without applying the compound of interest.

In another particular embodiment, the compound as defined in the section *"Compounds"* above is for maintaining synapses rate.

Another object of the present invention concerns a method for maintaining synapses rate in a subject suffering from, or at risk of suffering from a neurodegenerative disease as defined in the section *"Neurodegenerative disease"* above, comprising administering to said subject a therapeutically efficient amount of a compound as defined in the section *"Compounds"* above.

By "synapses rate" is meant herein the ratio of the number of synapses and the number of neurons.

By "maintaining synapses rate" is meant herein that the synapses rate is similar (*i.e.* not statistically significantly different) when the compound of interest is applied compared to the synapses rate observed in control conditions, such as same conditions but without applying the compound of interest or conditions where the neurons are not faced to neurodegenerative conditions.

In another particular embodiment, the compound as defined in the section *"Compounds"* above is for promoting formation of new synapses.

Another object of the present invention concerns a method for promoting formation of new synapses in a subject suffering from, or at risk of suffering from a neurodegenerative disease as defined in the section *"Neurodegenerative disease"* above, comprising administering to said subject a therapeutically efficient amount of a compound as defined in the section *"Compounds"* above.

By "promoting formation of new synapses" is meant herein that the number of newly formed synapses is higher, preferably statistically significantly higher, when the compound of interest is applied compared to the number of newly formed synapses observed in the same conditions but without applying the compound of interest. Preferably, the formation of new synapses is increased of at least 120% compared to formation of new synapses observed in the same conditions but without applying the compound of interest.

In another particular embodiment, the compound as defined in the section *"Compounds"* above is for decreasing Tau hyperphosphorylation.

Another object of the present invention concerns a method for decreasing Tau phosphorylation in a subject suffering from, or at risk of suffering from a neurodegenerative disease as defined in the section *"Neurodegenerative disease"* above, comprising administering to said subject a therapeutically efficient amount of a compound as defined in the section *"Compounds"* above.

By "Tau hyperphosphorylation" is meant herein the abnormally high phosphorylation of the Tau protein, in particular the abnormal phosphorylation of the Tau protein at at least one of the Thr39, Ser46, Thr50, Ser68, Thr69, Thr71, Ser113, Thr153, Thr175, Thr181, Ser184, Ser185, Ser191, Ser198, Ser199, Ser202, Thr205, Ser208, Ser210, Thr212, Ser214, Thr217, Thr231, Ser235, Ser237, Ser238, Ser241, Ser258, Ser262, Ser285, Ser289, Ser305, Ser324, Ser352, Ser356, Tyr394, Ser396, Ser400, Thr403, Ser404, Ser409, Ser412, Ser413, Thr414, Ser416, Ser422, Ser433 and Ser435 residues.

By "decreasing Tau hyperphosphorylation" is meant herein that at least one of the Thr39, Ser46, Thr50, Ser68, Thr69, Thr71, Ser113, Thr153, Thr175, Thr181, Ser184, Ser185, Ser191, Ser198, Ser199, Ser202, Thr205, Ser208, Ser210, Thr212, Ser214, Thr217, Thr231, Ser235, Ser237, Ser238, Ser241, Ser258, Ser262, Ser285, Ser289, Ser305, Ser324, Ser352, Ser356, Tyr394, Ser396, Ser400, Thr403, Ser404, Ser409, Ser412, Ser413, Thr414, Ser416, Ser422, Ser433 and Ser435 residues, in particular one of Ser212 and Thr214 residues of the Tau protein is not abnormally phosphorylated when the compound of interest is applied whereas said residue is abnormally phosphorylated in the same conditions but without applying the compound of interest.

By "therapeutically effective amount" is meant herein sufficient amount of the compound to act as neuroprotective agent in the treatment and/or prevention of a neurodegenerative disease at a reasonable benefit/risk ratio applicable to any medical treatment. It will be understood, however, that the total daily usage of the compounds of the present invention will be decided by the attending physician within the scope of sound medical judgment. The specific therapeutically effective dose level for any particular subject will depend upon a variety of factors including the disorder being treating and the severity of the disorder, activity of the specific compounds employed, the age, body weight, general health, sex and diet of the subject, the time of administration, route of administration and rate of excretion of the specific compounds employed, the duration of the treatment, drugs used in combination or coincidental with the specific compounds employed, and like factors well known in the medical arts. For example, it is well within the skill of the art to start doses of the compounds at levels lower than those required to achieve the desired therapeutic effect and to gradually increase the dosage until the desired effect is achieved.

The compounds used in the context of the invention may be administered in the form of a pharmaceutical composition including pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The form of the pharmaceutical compositions including the compounds used in the context of the invention and the route of administration naturally depend upon the condition to be treated, the severity of the illness, the age, weight, and gender of the patient, etc.

The compounds used in the context of the invention can be formulated for a topical, oral, parenteral, intranasal, intravenous, intramuscular, subcutaneous or intraocular administration and the like. In a particular embodiment, the compound used in the context of the invention is administered orally.

Alternatively, the pharmaceutical compositions including the compounds used in the context of the invention may contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

To prepare pharmaceutical compositions, an effective amount of the compounds used in the context of the invention may be dissolved or dispersed in a pharmaceutically acceptable carrier or aqueous medium.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

The carrier can be a solvent or dispersion medium containing, for example, water, ethanol, polyol (for example, glycerol, propylene glycol, and liquid polyethylene glycol, and the like) and suitable mixtures thereof. The proper fluidity can be maintained, for example, by the use of a coating, such as lecithin, by the maintenance of the required particle size in the case of dispersion and by the use of surfactants, stabilizing agents, cryoprotectants or antioxidants. The prevention of the action of microorganisms can be brought about by antibacterial and antifungal agents. In many cases, it will be preferable to include isotonic agents, for example, sugars or sodium chloride.

Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 mL of isotonic NaCl solution and either added to 1000 mL of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. The person responsible for administration will, in any event, determine the appropriate dose for the individual subject.

The compound used in the context of the invention can typically be administered orally for example in the form of tablets, capsules, microcapsules, powders, granules, syrups, solutions or suspensions taken by the oral or sublingual route.

In a particular embodiment, the compound used in the context of the invention is suitably administered orally at a daily dose of 1 to 20 mg/kg of body weight, in particular at a daily dose of 2 mg/kg.

In another particular embodiment, the compounds used in the context of the invention are suitably administered orally, twice a week, at a dose of 1 to 20 mg/kg of body weight, in particular at a dose of 1 mg/kg twice a week.

According to another particular embodiment, the compound used in the context of the invention is administered at doses of 10 to 1000 mg per day, preferably at doses of 0.2 to 2 mg 5 times a day.

The present invention will be further illustrated by the examples below.

### Examples

### Example 1

This example shows the effect of Donecopride in rat primary hippocampal neurons injured with Aβ (24h exposure).

Alzheimer disease (AD) is a neurodegenerative disease which affects mainly people over the age of 65, suffering from different clinical symptoms such as progressive decline in thinking, language, and learning capacity. Increasing evidence from various sources points to Aβ Oligomers (AβO)/protofibrils as putative toxic species in AD pathogenesis.

By generating a solution of AβO and playing on the concentration of and time of exposure to AβO, it was possible to reproduce early effects (oxidative stress) and the long-term development of structural alterations (death of neurons).

The model used in the present example, using Aβ peptide solution containing AβO (precisely measured by automatic Western Blot), reproduced essential neuropathological features of AD.

The aim of this study was to evaluate the effects of one test compound (Donecopride fumarate at several concentrations) in rat primary hippocampal neurons injured with Aβ (24h exposure). Donepezil of the following formula : 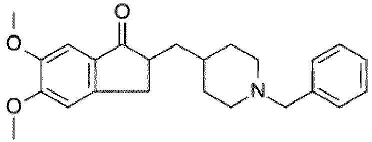 (one concentration) was used as reference test compound.

### Materials and methods

### Primary culture of hippocampal neurons

Rat hippocampal neurons were cultured as described by Callizot et al. (2013) J. Neurosci. Res. 91706-716. Pregnant female rats of 17 days gestation (Rats Wistar; Janvier Labs France) were killed using a deep anesthesia with CO₂ chamber followed by a cervical dislocation. Then, fetuses were removed from the uterus and immediately placed in ice-cold L15 Leibovitz medium with a 2% penicillin (10,000 U/ml) and streptomycin (10 mg/ml) solution (PS) and 1% bovine serum albumin (BSA). Hippocampal neurons were treated for 20 min at 37°C with a trypsin-EDTA solution at a final concentration of 0.05% trypsin and 0.02% EDTA. The dissociation was stopped by adding Dulbecco's modified Eagle's medium (DMEM) with 4.5 g/l of glucose, containing DNAse I grade II (final concentration 0.5 mg/ml) and 10% fetal calf serum (FCS). Cells were mechanically dissociated by three forced passages through the tip of a 10-ml pipette and then centrifuged at 515 x g for 10 min at 4°C. The supernatant was discarded, and the pellet was resuspended in a defined culture medium consisting of Neurobasal medium with a 2% solution of B27 supplement, 2 mmol/l of L-glutamine, 2% of PS solution, and 10 ng/ml of brain-derived neurotrophic factor (BDNF). Viable cells were counted in a Neubauer cytometer, using the trypan blue exclusion test. Cells were seeded at a density of 20,000 per well in 96-well plates precoated with poly-L-lysine and cultured at 37°C in an air (95%)-CO₂ (5%) incubator. The medium was changed every 2 days.

### Donecopride and human Aβ1-42 exposure

The hippocampal neurons were exposed with Aβ solutions (see below) after 17 days of culture. The Aβ1-42 preparation was done following the procedure described by Callizot et al. (2013) J. Neurosci. Res. 91706-716. Briefly, Aβ1-42 peptide was dissolved in the defined culture medium mentioned above, at an initial concentration of 40 µM. This solution was gently agitated for 3 days at 37°C in the dark and immediately used after being properly diluted in culture medium to the concentrations used (20 µM (plate 1) or 2.5 µM (plate 2) corresponding to 2 µM or 0.25 µM of oligomers (AβO) respectively).

Donecopride fumarate was solved in culture medium and preincubated 1 h before Aβ application. Aβ1-42 preparation was added to a final concentration of 20 or 2.5 µM (= 2 µM or 0.25 µM of AβO, evaluated by automatic Western Blot) diluted in control medium in presence of Donecopride.

### Organization of culture plates

Donecopride fumarate (Donecopride) was tested on one culture in a 96 well plate (6 wells per conditions). The compound was preincubated one hour before Aβ application. The following conditions were assessed:

| PLATE 1 (MAP-2 / Tau) | PLATE 2 (PSD95 / SYN) |
|---|---|
| Control (Vehicle) | Control (Vehicle) |
| + Aβ (20 µM 24 h) / vehicle | + Aβ (2.5 µM 24 h) / vehicle |
| + Aβ (20 µM 24 h) / Donecopride (1 µM) | + Aβ (2.5 µM 24 h) / Donecopride (1 µM) |
| + Aβ (20 µM 24 h) / Donecopride (500 nM) | + Aβ (2.5 µM 24 h) / Donecopride (500 nM) |
| + Aβ (20 µM 24 h) / Donecopride (100 nM) | + Aβ (2.5 µM 24 h) / Donecopride (100 nM) |
| + Aβ (20 µM 24 h) / Donecopride (50 nM) | + Aβ (2.5 µM 24 h) / Donecopride (50 nM) |
| + Aβ (20 µM 24 h) / Donecopride (10 nM) | + Aβ (2.5 µM 24 h) / Donecopride (10 nM) |
| + Aβ (20 µM 24 h) / Donecopride (5 nM) | + Aβ (2.5 µM 24 h) / Donecopride (5 nM) |
| + Aβ (20 µM 24 h) / Donecopride (1 nM) | + Aβ (2.5 µM 24 h) / Donecopride (1 nM) |
| + Aβ (20 µM 24 h) / Donepezil (1 µM) | + Aβ (2.5 µM 24 h) / Donepezil (1 µM) |

### End point evaluation

### Plate 1: SURVIVAL, NEURITE NETWORK AND PHOSPHO TAU EVALUATION

24 hours after intoxication, the hippocampal neurons were fixed by a cold solution of ethanol (95%) and acetic acid (5%) for 5 min at -20°C. After permeabilization with 0.1% of saponin, cells were incubated for 2 h with:
a) chicken polyclonal antibody anti microtubule-associated-protein 2 (MAP-2), diluted at 1/1000 in PBS containing 1% fetal calf serum and 0.1% of saponin (this antibody allows the specific staining of neuronal cell bodies and neurites; and allow the study of neuronal cell death and neurite network).
b) mouse monoclonal antibody anti-phospho Tau (AT100) at dilution of 1/400 in PBS containing 1% fetal calf serum and 0.1% of saponin.

These antibodies were revealed with Alexa Fluor 488 goat anti mouse IgG and Alexa Fluor 568 goat anti-chicken IgG at the dilution 1/400 in PBS containing 1% FCS, 0.1% saponin, for 1 h at room temperature.

For each condition, 30 pictures (representative of the whole well area) per well were taken using ImageXpress (Molecular Devices) at 20x magnification. All images were taken with the same acquisition parameters. Analyses were performed automatically by using Custom Module Editor (Molecular Devices).

The following endpoints were assessed:
- Total number of neurons (neuron survival, number of MAP-2 positive neurons)
- Neurite network (in µm of MAP-2 positive neurons)
- area of tau in neurons (µm², overlapping with MAP-2 positive neurons).

### Plate 2: SYNAPSES EVALUATION

24 hours after intoxication, the hippocampal neurons were fixed by a cold solution of ethanol (95%) and acetic acid (5%) for 5 min at -20°C. After permeabilization with 0.1% of saponin, cells were incubated for 2 h with:
a) mouse monoclonal antibody anti post synaptic density 95kDa (PSD95) at a dilution of 1/100 in PBS containing 1% fetal calf serum and 0.1% of saponin.
b) Rabbit polyclonal antibody anti-synaptophysin (SYN) at the dilution of 1/100 in PBS containing 1% fetal calf serum and 0.1% of saponin.

These antibodies were revealed with Alexa Fluor 488 goat anti mouse IgG and Alexa Fluor 568 goat anti-rabbit IgG at the dilution 1/400 in PBS containing 1% FCS, 0.1% saponin, for 1 h at room temperature.

For each condition, 40 pictures per well were taken using ImageXpress (Molecular Devices) with 40x magnification. All images were taken with the same acquisition parameters.

Synapses evaluation was performed automatically by using Custom module editor (Molecular Devices).

The following endpoints were assessed:
- Total number of synapses (overlapping between PSD95/SYN).

### Statistical analysis

All values are expressed as mean +/- SEM. Statistical analysis was performed by one-way ANOVA, followed by PLSD Fisher's test, p < 0.05 were considered significant.

Graphs and statistical analyses on the different conditions were performed using GraphPad Prism software version 7.04. *p<0.05 was considered significant.

### Results

*Effects of Donecopride on the survival and neurite network of MAP-2 hippocampal neurons and on the hyperphosphorylation of Tau.*

### Neuronal survival

The effect of Donecopride on the survival of MAP-2 neurons in a primary culture of hippocampal neurons is shown in Table 1 below.

**Table 1: MAP-2 neurons (% of control)**

| | **MAP-2 neurons (% of control)** | | |
|---|---|---|---|
| | **mean** | **sem** | **n** |
| Control | 100 | 5 | 6 |
| + Aβ 1-42 (20 µM) | 70 | 2 | 6 |
| + Donecopride (1 nM) | 70 | 2 | 5 |
| + Donecopride (5 nM) | 69 | 2 | 6 |
| + Donecopride (10 nM) | 73 | 2 | 5 |
| + Donecopride (50 nM) | 74 | 2 | 5 |
| + Donecopride (100 nM) | **78*** | 1 | 4 |
| + Donecopride (500 nM) | **79*** | 1 | 5 |
| + Donecopride (1 µM) | **62*** | 2 | 5 |
| + Donepezil (1 µM) | **82*** | 2 | 5 |

| | | | |
|---|---|---|---|
| * : p < 0.05 versus Aβ1-42, determined by one-way ANOVA followed by a Fisher's LSD test. | | | |

An important loss of MAP-2 neurons was consecutive to the application of the Aβ peptide. Donepezil, used here as a positive control, was able to significantly protect neurons from death. Two doses of Donecopride (100 nM and 500 nM) exerted neuroprotective effects to a similar extent to Donepezil.

### Neurite network

The effect of Donecopride on the neurite network of MAP-2 neurons in a primary culture of hippocampal neurons is shown in Table 2 below.

**Table 2: MAP-2 neurite network (% of control)**

| | **MAP-2 neurite network (% of control)** | | |
|---|---|---|---|
| | **mean** | **sem** | **n** |
| Control | 100 | 2 | 6 |
| + Aβ 1-42 (20 µM) | 63 | 1 | 6 |
| + Donecopride (1 nM) | 64 | 2 | 5 |
| + Donecopride (5 nM) | 69 | 2 | 6 |
| + Donecopride (10 nM) | 68 | 2 | 5 |
| + Donecopride (50 nM) | **71*** | 3 | 5 |
| + Donecopride (100 nM) | **73*** | 1 | 5 |
| + Donecopride (500 nM) | **79*** | 3 | 6 |
| + Donecopride (1 µM) | **76*** | 2 | 6 |
| + Donepezil (1 µM) | **92*** | 3 | 5 |

| | | | |
|---|---|---|---|
| * : p < 0.05 versus Aβ1-42, determined by one-way ANOVA followed by a Fisher's LSD test. | | | |

Total neurite network was severely shrunk upon Aβ 1-42 injury. Donepezil has significantly preserved almost all the neurite network. Four doses of Donecopride (50 nM, 100 nM, 500 nM and 1µM) exerted also protective effects on the neurite network.

The neurite network is proportional to the number of neurons in the culture. The total neurite network was normalized by the number of neurons, in order to determine the average length of neurite per neurons. The corresponding results are shown in Table 3 below.

**Table 3: Neurite network / MAP-2 neurons (% of control)**

| | **Neurite network / MAP-2 neurons (% of control)** | | |
|---|---|---|---|
| | **mean** | **sem** | **n** |
| Control | 100 | 6 | 6 |
| + Aβ 1-42 (20 µM) | 90 | 3 | 6 |
| + Donecopride (1 nM) | 90 | 3 | 4 |
| + Donecopride (5 nM) | 99 | 2 | 6 |
| + Donecopride (10 nM) | 96 | 3 | 4 |
| + Donecopride (50 nM) | 91 | 5 | 4 |
| + Donecopride (100 nM) | 93 | 1 | 4 |
| + Donecopride (500 nM) | 100 | 4 | 5 |
| + Donecopride (1 µM) | **124*** | 5 | 5 |
| + Donepezil (1 µM) | **113*** | 4 | 4 |

| | | | |
|---|---|---|---|
| * : p < 0.05 versus Aβ1-42, determined by one-way ANOVA followed by a Fisher's LSD test. | | | |

This adjustment showed that neurite length per neuron was longer in presence of Donepezil and Donecopride (1µM), suggesting that these two compounds had an effect on neurite outgrowth.

### Tau phosphorylation

The effect of Donecopride on the phosphorylation of Tau (AT100) in MAP-2 neurons in a primary culture of hippocampal neurons is shown in Table 4 below.

**Table 4: Area of Tau (AT100)/neurons (% of control)**

| | **Area of Tau (AT100)/neurons (% of control)** | | |
|---|---|---|---|
| | **mean** | **sem** | **n** |
| Control | 100 | 4 | 5 |
| + Aβ 1-42 (20 µM) | 200 | 11 | 5 |
| + Donecopride (1 nM) | 204 | 7 | 4 |
| + Donecopride (5 nM) | 201 | 8 | 5 |
| + Donecopride (10 nM) | 175 | 11 | 4 |
| + Donecopride (50 nM) | **161*** | 12 | 5 |
| + Donecopride (100 nM) | **154*** | 6 | 3 |
| + Donecopride (500 nM) | **152*** | 8 | 4 |
| + Donecopride (1 µM) | 198 | 8 | 5 |
| + Donepezil 1 µM) | **151*** | 6 | 4 |

| | | | |
|---|---|---|---|
| * : p < 0.05 versus Aβ1-42, determined by one-way ANOVA followed by a Fisher's LSD test. | | | |

An hyperphosphorylation of Tau (on AT100) was observed under application of Aβ 1-42. Donepezil was able to significantly mitigate this hyperphosphorylation. Similarly, Donecopride (50 nM, 100 nM, 500 nM) has significantly reduced the hyperphosphorylation of Tau on a dose-dependent manner.

### Effects of Donecopride on synapses in a primary culture of hippocampal neurons

To assess the formation of synapses, the distribution of PSD-95, a post-synaptic marker, and synaptophysin, a pre-synaptic marker, was studied. A structure positive for both PSD-95 and synaptophysin staining was considered as a synapse.

The effect of Donecopride on these synapses is shown in Table 5 below.

**Table 5: Number of synapses (% of control)**

| | **Number of synapses (% of control)** | | |
|---|---|---|---|
| | **mean** | **sem** | **n** |
| Control | **100*** | 6 | 5 |
| + Aβ 1-42 (20 µM) | 67 | 4 | 6 |
| + Donecopride (1 nM) | 69 | 5 | 4 |
| + Donecopride (5 nM) | **83*** | 3 | 5 |
| + Donecopride (10 nM) | **87*** | 7 | 5 |
| + Donecopride (50 nM) | **91*** | 3 | 6 |
| + Donecopride (100 nM) | **93*** | 3 | 4 |
| + Donecopride (500 nM) | **94*** | 5 | 4 |
| + Donecopride (1 µM) | **85*** | 4 | 5 |
| + Donepezil (1 µM) | **88*** | 6 | 5 |

| | | | |
|---|---|---|---|
| * : p < 0.05 versus Aβ1-42, determined by one-way ANOVA followed by a Fisher's LSD test. | | | |

The stress resulting from the application of Aβ 1-42 caused a reduction in the number of synapses (as previously shown by Callizot et al. (2013) J. Neurosci. Res. 91 :706-716).

Donepezil mitigated the loss of synapses. Similarly, Donecopride was also able to preserve the number of synapses, on a dose dependent manner (from 5 nM to 1 µM). Interestingly, this effect was observed a low dose of the compound (5 nM). This effect increased with the dose.

Due to the injury caused by the Aβ 1-42 peptide, the number of neurons was reduced in the experimental conditions, which led to a reduction in the number of synapses. Therefore, to estimate the number of synapses per neuron, the number of synapses was normalized by the number of neurons for the control, Aβ 1-42, Donecopride (500 nM, the condition showing the highest number of synapses), and Donepezil groups.

This adjustment showed that ONLY DONECOPRIDE significantly promoted the formation of new synapses while Donepezil had no effect.

### Conclusion

The present study indicates that Donecopride has beneficial effects on the neuronal survival and neurite network of hippocampal neurons injured with Aβ 1-42 peptide, an *in vitro* model of Alzheimer disease.

Moreover, Donecopride was able to reduce the hyperphosphorylation of Tau on the site AT100 and was able to significantly stimulate the formation of new synapses.

The effects of Donecopride were greater than those of Donepezil, suggesting that this compound is an interesting drug candidate as neuroprotective agent, in particular for treating neurodegenerative diseases.

### Example 2

The present example demonstrates that the compounds of the invention can improve cognitive performances of mice in an AD/s *in vivo* model.

For that purpose, the efficacy of two compounds (Donecopride fumarate and Flucopride fumarate) was investigated using wild-type C57BI6/J mice challenged with an intracerebroventricular injection of Aβ1-42 oligomers (AβO).

Compounds were dosed per os (through oral gavage) at three different doses. Cognitive performances were assessed using the Y-maze test, the Novel Object Recognition assay and the Morris-water maze test. At the end of the cognitive trials, all animals were sacrificed, blood and brain sampled for further ex-vivo analyses.

### Schematic representation of the experiment

Briefly, 144 C57BI6/J male mice (3 months of age) were received and housed (4 to 5 per cage) in quarantine during 7 days, or longer. Test items were dosed per os (through oral gavage) once daily at different concentrations from day -1 to day +17.

At day 0, mice received a single icv injection of vehicle or AβO.

At day +4 (i.e. four days after disease induction), spatial working memory was assessed using the Y-maze test.

From days +3 to days +14, learning capacities and long-term memory were investigated in the MWM assay.

At days +15/+16, recognition memory was investigated using the Novel Object recognition test (NOR).

Animals were sacrificed at day +17 and tissues prepared for further ex-vivo analyses.

### Description of the experimental groups

The study involved a total of 144 mice divided in 12 experimental groups with 12 mice per experimental group. All animals received a single (and unilateral) icv injection of vehicle or AβO in a total volume of 1 µL.

The different experimental groups are defined as follow:
- **GROUP A (vehicle CTRL):** po vehicle AND icv injection of vehicle (n = 12)
- **GROUP B (AβO CTRL):** po vehicle AND icv injection of AβO (n = 12)
- **GROUP C (positive CTRL):** po Donepezil subchronic AND icv injection of AβO (n = 12)
- **GROUP D (Donecopride CTRL):** po Donecopride (9 mg/kg) AND icv injection of vehicle (n = 12)
- **GROUP E (Flucopride CTRL):** po Flucopride (9 mg/kg) AND icv injection of vehicle (n = 12)
- **GROUP F (Donecopride Treated):** po Donecopride (1 mg/kg) AND icv injection of AβO (n = 12)
- **GROUP G (Donecopride Treated):** po Donecopride (3 mg/kg) AND icv injection of AβO (n = 12)
- **GROUP H (Donecopride Treated):** po Donecopride (9 mg/kg) AND icv injection of AβO (n = 12)
- **GROUP I (Fluocopride Treated):** po Flucopride (1 mg/kg) AND icv injection of AβO (n = 12)
- **GROUP J (Flucopride Treated):** po Flucopride (3 mg/kg) AND icv injection of AβO (n = 12)
- **GROUP K (Flucopride Treated):** po Flucopride (9 mg/kg) AND icv injection of AβO (n = 12)
- **GROUP L (positive CTRL):** ip Donepezil acute on test days AND icv injection of AβO (n = 12)
Because a maximum of 24 mice could be icv injected per day, the study was performed in six independent runs. Two mice from all groups were included in each run.

### Experimental procedures

### Animal housing

Reception of 144 C57BI6/J male (from Janvier, France) mice (3 months of age). Upon receipt, animals were placed in quarantine for a period of 7 days, or longer.

Mice were housed at standard temperature (22 ± 2°C) and in a light- and humidity-controlled environment (lights on from 8 am to 8 pm; humidity 55 ± 10%) with ad libitum access to food and water. Mice were group housed and monitored twice-a-day by laboratory personnel (8 am and 4 pm). In case of 2 or more significant problems of the general health status, the affected mouse was sacrificed. Definitions of unacceptable health status are as follow: no spontaneous movements and inability to drink or feed during a 24-h observation period, more than 12 % weight loss, massive bleeding, spontaneous inflammation (general observations of mouse skin and eyes), or missing anatomy.

Over the entire procedure mice were weighted 3 times (i.e. before treatment and at days +8 and +17).

### Compound storage, formulation and dosing

For per os dosing, Donecopride and Flucopride were formulated by solubilization in 0.9% NaCl. Compound dosing (total volume of 200 µL) started one day before induction of the disease (through icv injection of AβO) and was done for 19 consecutive days.

### Stereotaxic injection

**Single intra-cerebroventricular injection of 144 mice with vehicle or AβO (max. 24 mice per day, all mice randomized):** under anesthesia (ip injection of a mixture of ketamine/xylasine at a dose of 110 and 15 mg/kg, respectively) AβO (1 µL), or vehicle (1 µL) were injected into the right lateral ventricle. Injections were made using 10 µL Hamilton micro syringes fitted with a 26-gauge needle. The procedure was terminated by a subcutaneous injection of metacam (analgesia) at a dose of 5 mg/kg. Animals were then placed individually in their home-cage and the cage was placed in a heated cabinet until the animal has fully recovered. Animals were carefully monitored to control recovery after anesthesia.

**Preparation of AβO:** Aβ1-42 was obtained from Bachem (ref H1368) (batch number: 1052301). The preparation of stable Aβ1-42 oligomers was performed as described in Garcia et al. (2010) J. Neurosci. 30:7516-7527. The oligomeric preparation (identified as Batch#2016-14) contained a mixture of stable trimers and tetramers of Aβ1-42, as well as monomeric forms of the peptide. All oligomer preparations used were previously characterized in terms of oligomer composition and *in vitro* neurotoxicity.

### Cognition test, Y-maze test

6 independent trials involving each 24 mice (i.e. 2 mice from each experimental group) for a total of 144 mice at day +4 - Behavioral tests were performed 1 h after dosing.

This test assesses spatial working memory which is mainly mediated by the prefrontal cortex (working memory) and the hippocampus (spatial component). See Yoon et al. (2008) Learn Mem. 15:97-105 and Spellman et al. (2015) Nature 522:309-314 for the different components.

Prefrontal cortex mediates working memory in rodent, primates and human.

The measurement of spontaneous alternation behavior has been widely used by behavioral pharmacologists to assess spatial working memory, a component of short-term memory. In its simplest form, spontaneous alternation behavior comprises the tendency of mice to alternate their conventional non reinforced choice of arms on successive opportunities.

This test has been successfully implemented using icv infusion of AβO to induce cognitive deficits (Garcia et al. (2010) J. Neurosci. 30:7516-7527).

**Detailed protocol:** Immediate spatial working memory performance was assessed by recording spontaneous alternation behavior in a Y-maze. The maze is made of opaque Plexiglas and each of the three arms is 40 cm long, 14 cm high, 10 cm wide and positioned at equal angles. The apparatus is placed in a homogeneously lit test room to obtain 12-15 lux in all arms as well as in the central zone. Mice are placed in the middle of one arm and allowed to explore the maze freely during 5 min session. The series of arm entries are video recorded (Smart v3.0 software, Bioseb) and an arm entry is considered complete when the hind paws of the mouse are completely placed in the arm. Alternation is defined as successive entries into the 3 arms on overlapping triplet sets. The percentage of alternation is calculated as the ratio of actual total alternations to possible alternations, defined as the number of arm entries minus 2, multiplied by 100. Locomotor activity is also recorded and evaluated during this phase (e.g. motivational index) by monitoring average speed and total distance.

**Data analysis:** Graphpad/Prism computer software is used for the statistical analyses. A non-parametric analysis of variance (Kruskal Wallis test) was carried out followed by non-parametric Mann-Whitney U tests to compare between groups. Values with p<0.05 are considered statistically significant. Data are presented as mean ± SEM.

### Cognition testing, NOR assay

6 independent trials involving each 24 mice (i.e. 2 mice from each experimental group) for a total of 144 mice at day +4 - Behavioral tests were performed 1 h after dosing.

This test assesses recognition memory for items and its human equivalent is the visual pairwise-comparison (VPC) (Wallace et al. (2015) Handb. Exp. Pharmacol. 228:27-57). Recognition for object is mediated by the perirhinal cortex in rodents (Albasser et al. (2009) Behav. Neurosci. 123:115-124), primates (Zeamer et al. (2015) Dev. Cogn. Neurosci. 11:31-41) and humans (Watson & Lee (2013) J. Neurosci. 33:4192-4200).

It was previously demonstrated that intra-cerebro-ventricular injection of AβO to mice leads to an impairment in novel object recognition task and is reversible with the acetylcholine esterase inhibitor donepezil and showed protective effect with human in a cytoprotective and neuroprotective mitochondrial-derived peptide.

**Detailed protocol:** One day before the cognitive test (i.e. at day +15), mice are habituated during a 10 minutes' trial during which they are placed in an empty open field. The day of the cognitive test (i.e. day +16), animals are placed in the same open field and are allowed to explore freely two identical objects for a trial of five minutes (acquisition trial). Then the animals are returned in their home-cage for an inter-trial time of five minutes. During the retention trial, animals are allowed to explore two different objects: one familiar and one novel object. During this time, the experimenter, blind to the treatment, record the time the mouse is actively exploring each object. All trials are video recorded (Smart v3.0 software, Bioseb). A discrimination index is then generated: Discrimination index = (time exploring novel object - time exploring familiar object) / total exploration time.

**Data analysis:** Graphpad/Prism computer software is used for the statistical analyses. A non-parametric analysis of variance (Kruskal Wallis test) is carried out followed by non-parametric Mann-Whitney U tests in order to compare between groups. Values of p<0.05 are considered statistically significant. Data are presented as mean ± SEM.

### Cognition testing, MWM assay

6 independent trials involving each 24 mice (i.e. 2 mice from each experimental group) for a total of 144 mice at day +4.

Spatial reference memory is a process, which tests the ability to remember the spatial cues to identify a location. It is mediated by the hippocampus in rodents (Broadbent et al. (2006) Learn Mem. 13:187-191) and in human (Bartsch et al. (2010) Science 328:1412-1415) using a virtual water-maze task. This test also involves a learning component.

Injection of AβO icv in mice induces a deficit in spatial reference memory in the Morris Water Maze (Garcia et al. (2010) J. Neurosci. 30:7516-7527).

### Detailed protocol:

Habituation trials (visible platform) - The Morris water maze (MWM) was performed as described in Garcia et al. (2010) J. Neurosci. 30:7516-7527. The experimental apparatus consists of a circular water tank (diameter = 100 cm; height = 50 cm) containing water at 21°C to a depth of 25 cm and rendered opaque to block the view past the water surface by adding an aqueous acrylic emulsion. A platform (diameter = 10 cm) is used and placed at the midpoint of a quadrant. The pool is located in a test room, homogeneously illuminated at 100 lux. The swimming path of the animals is recorded using a video tracking system. Mice are brought to the experimental room for at least 30 min prior to testing, to enable acclimation to the experimental room conditions. Navigation to a visible platform is carried out before place-navigation, to evaluate visual and motor abilities of all mice. Mice are submitted to 4 trials of 60 s per day (during 2 consecutive days), with an inter-trial interval of at least 1 h. Once mice have found the platform, they are left alone on the platform for an additional time of 30 s. There are no additional cues in the room. The platform position and starting points are randomly distributed over all 4 quadrants of the pool. Mice that fail to find the platform after 60 s are guided to its location and placed on the platform for 30 s.

Memory-acquisition trials (learning trials with hidden platform) - They are performed during 5 consecutive days and used to reach a steady state of escape latency. Mice are brought to the experimental room for at least 30 min prior to testing, to enable acclimation to the experimental room conditions. The hidden platform is submerged 1 cm below the water surface and placed at the midpoint of one quadrant. The pool is located in a test room homogeneously illuminated at 100 lux and containing various prominent visual cues. The swimming paths, swimming distance, swimming speed and thigmotaxis are recorded using a video tracking system. Mice are submitted to 4 trials of 60 s per day, with an inter-trial interval of at least 1 h. The mice are allowed to swim freely for 60 s, left alone for an additional 30 s on the hidden platform and then returned to their home cage during the inter-trial interval. Start positions (set at the border of quadrants) are randomly selected for each animal. In each trial, the time required to escape onto the hidden platform is recorded. Mice that fail to find the platform after 60 s are guided to and placed onto the platform for 30 s, before they are returned to their home cage.

Memory-retention trials (probe trials, no platform) - They are performed two days after the last training session. Mice are again acclimated to the experimental room for at least 30 min prior to testing. The platform is removed and each animal is allowed a free 60 sec swim During the trial, the time spent in the target quadrant, the time spend in the quadrant opposite, and the crossings over the former platform location are measured and monitored by video tracking.

**Data analysis:** Graphpad/Prism computer software is used for the statistical analyses. A non-parametric analysis of variance (Kruskal Wallis test) is carried out followed by non-parametric Mann-Whitney U tests in order to compare between groups. Values of p<0.05 are considered statistically significant. Data are presented as mean ± SEM.

### Tissue sampling

Mice were sacrificed on day +17.

**Blood sample collection:** under anesthesia, mice are bled out. Approximately 600 µL of whole blood was taken from the vena cava with a 1 mL syringe. Blood was collected in BD Microtainer K2E tubes (ref. 365975) or Sarstedt LiHe microtubes (ref. 41.1503.005). The tubes were gently inverted and samples placed on ice. Microtainers were centrifuged for 5 min at 4°C, 8600 g. Approximately 250 µL plasma were transferred in Eppendorf tubes and frozen at -80°C for further analyses.

**Brain sample collection:** following blood collection, mice were intra-cardiac flash-perfused with 0.9% saline. The whole brain was removed and hemispheres were separated. The contralateral left hemisphere was flash-frozen in liquid nitrogen and stored at -80°C for further analysis (i.e. compound exposure). The ipsilateral right hemisphere was used to isolate hippocampus, pre-frontal cortex, and cortex. All brain structures were isolated on cooled support and flash-frozen in liquid nitrogen.

### Results

### Effects of the compounds of the invention on spatial working memory

To determine the effect of Donecopride and Flucopride on spatial working memory of mice, a Y-maze test was performed four days after icv injection of either vehicle or AβO. During the 5 min trial animals were allowed to explore the Y-maze device and alternation behavior was scored as described in the Experimental Procedures.

Mice from the vehicle control group (Group A) exhibited normal behavior in the exploration of the Y-maze with an alternation behavior of 68.0 ± 3.0 %. These results are in agreement with previous observations of similar control groups (see Garcia et al. (2010) J. Neurosci. 30:7516-7527).

As expected, a single icv injection of AβO (Group B) resulted in a significant impairment (p=0.0026) of the cognitive performances in the Y-maze test when compared to vehicle control mice, with only 54.0 ± 2.0 % alternations.

Positive control mice, dosed per os with Donepezil and icv injected with AβO (Group C) were indistinguishable from control mice (alternation behavior of 66.1 ± 1.8 %) and statistically different (p=0.0011) from AβO-injected mice. Positive control mice, dosed ip (acutely) with donepezil and icv injected with AβO (Group L) were statistically different from control mice (p=0.0278; alternation behavior of 60.8 ± 1.9 %) and also statistically different (p=0.0421) from AβO-injected mice.

Mice dosed with Donecopride (9 mg/kg/day) and icv injected with vehicle (Group D) exhibited an alternation behavior of 59.3 ± 4.0 %, not different from control mice (p=0.2640) and not different from AβO-injected mice (p=0.5371).

Mice dosed with Flucopride (9 mg/kg/day) and icv injected with vehicle (Group E) exhibited an alternation behavior of 63.1 ± 2.3 %, not different from control mice (p=0.1166) and different from AβO-injected mice (p=0.0111).

Mice were dosed with increasing doses of Donecopride (1, 3 and 9 mg/kg/day) and were icv injected with AβO (Groups F, G and H, respectively). At a dose of 3 mg/kg/day, Donecopride inhibited AβO-induced impairment of spatial working memory. Indeed, mice from Group G exhibited an alternation behavior of 65.5 ± 4.4 %, not different from control mice (p=0.5543) and different from AβO-injected mice (p=0.0137). At a dose of 1 mg/kg/day, Donecopride had no effect on AβO-induced impairment of spatial working memory as these mice exhibited an alternation behavior of 56.6 ± 3.7 %, different from control mice (p=0.0488) and not different from AβO-injected mice (p=0.6859). Due to a higher heterogeneity, mice from Group H (dosed with Donecopride at 9 mg/kg/day) exhibited an intermediate phenotype with an alternation behavior of 64.3 ± 5.1 %, not different from control mice (p=0.5994) and also not different from AβO-injected mice (p=0.1316).

Mice were dosed with increasing doses of Flucopride (1, 3 and 9 mg/kg/day) and were icv injected with AβO (Groups I, J and K, respectively). At doses of 1 and 3 mg/kg/day, Flucopride inhibited AβO-induced impairment of spatial working memory. Indeed, mice from Groups I and J exhibited an alternation behavior of 67.1 ± 2.7 % and 65.3 ± 4.1 %, respectively, not different from control mice (p=0.9754 and p=0.6936, for groups I and J, respectively) and different from AβO-injected mice (p=0.0029 and p=0.0454, for groups I and J, respectively). In contrast, at a dose of 9 mg/kg/day, Flucopride failed to protect mice from AβO-induced impairment of spatial working memory. Indeed, mice from Group K exhibited an alternation behavior of 59.1 ± 3.0 %, different from control mice (p=0.0290) but not different from AβO-injected mice (p=0.2346).

### Effects of the compounds of the invention on learning capacities and long-term memory

To determine the effect of the compounds of the invention on learning capacities and long-term memory, a MWM test was performed from day +3 to day +14 after icv injection of either vehicle or AβO.

### Habituation trials

In a first step (habituation trials performed at Days +3 and +4 after icv infusion of vehicle or AβO) mice were trained to escape from the water to a visible platform in the absence of other visual cues. During these trials, escape latency, swimming distance and swimming speed were recorded. There was no significant difference between groups in escape latency. No difference in swimming speed or swimming distance was detected.

Altogether, these data suggest that treatment did not affect the physical capacity nor the motivation of mice to escape from water.

### Learning trials

The learning capacities of mice was monitored during five consecutive days (from day +7 to day +11 after icv infusion of vehicle or AβO). Animals performed 4 trials per day and were trained to localize a hidden platform using visual cues. During these learning trials, escape latency, swimming distance and swimming speed were recorded.

There was no statistically significant difference in the mean escape latency between groups at the beginning of the trials. All groups then appeared to exhibit some degree of learning.

Mice from the vehicle control group (Group A) learned efficiently the platform localization, and after five days of training (day 5), they exhibited a mean escape latency of 11.7 ± 1.5 s (p=0.00031 as compared to day 1). In contrast, the AβO-injected mice (Group B) exhibited an impaired behavior resulting in a mean escape latency of 17.3 ± 2.0 s after five days of training (p=0.4537, not different from day 1 and p=0.00293, significantly different from control mice).

Mice dosed sub-chronically with donepezil (po) and icv injected with AβO (Group C) learned efficiently the platform localization. After five days of training (day 5), they exhibited a mean escape latency of 14.7 ± 1.9 s (p=0.09593 as compared to day 1). At day 5, their performances were not different from control mice (p=0.2188) and not different from AβO-injected mice (p=0.3477).

Mice dosed acutely (on the test day) with donepezil (ip) and icv injected with AβO (Group L) learned efficiently the platform localization. After five days of training (day 5), they exhibited a mean escape latency of 13.4 ± 1.5 s (p=0.01573 as compared to day 1). At day 5, their performances were not different from control mice (p=0.4187) and not different from AβO-injected mice (p=0.1272).

Mice dosed with Donecopride (9 mg/kg/day) or Flucopride (9 mg/kg/day) and icv injected with vehicle (Groups D and E, respectively) exhibited normal cognitive performances. After five days of training (day 5), mice dosed with Donecopride exhibited a mean escape latency of 13.0 ± 1.7 s (p=0.01778 as compared to day 1). At day 5, their performances were not different from control mice (p=0.5476) and not different from AβO-injected mice (p=0.10731). After five days of training (day 5), mice dosed with Flucopride exhibited a mean escape latency of 16.3 ± 2.0 s (p=0.04439 as compared to day 1). At day 5, their performances were (for unknown reasons) different from control mice (p=0.06709) and not different from AβO-injected mice (p=0.7235). It is worthy to note that at day 4, Flucopride exhibited a mean escape latency of 14.2 ± 1.9 s, not different from control mice (p=0.7933) and not different from AβO-injected mice (p=0.1225).

Mice were dosed with increasing doses of Donecopride and icv injected with AβO (Groups F, G and H). The presence of Donecopride (at a dose of 3 mg/kg/day) resulted in an improvement of learning capacities of mice as compared with AβO-injected mice. Indeed, mice from group G exhibited a mean escape latency of 12.7 ± 1.5 s (p=0.02933 as compared to day 1) after five days of training. Their performances were not different from control mice (p=0.6284) but different from AβO-injected mice (p=0.07381).

Mice were dosed with increasing doses of Flucopride and icv injected with AβO (Groups I, J and K). The presence of Flucopride (at a dose of 1 and 3 mg/kg/day) resulted in an improvement of learning capacities of mice as compared with AβO-injected mice, whereas the compound at a dose of 9 mg/kg/day had no effect. Mice dosed with Flucopride at 1 mg/kg/day exhibited a mean escape latency of 13.1 ± 1.1 s (p=0.03759 as compared to day 1) after five days of training. Their performances were not different from control mice (p=0.4367) but different from AβO-injected mice (p=0.07422). Similarly, mice dosed with Flucopride at 3 mg/kg/day exhibited a mean escape latency of 12.5 ± 1.1 s (p=0.00146 as compared to day 1) after five days of training. Their performances were not different from control mice (p=0.6477) but different from AβO-injected mice (p=0.04328).

### Probe trials

Long-term memory performance was determined four days after the last training session and 14 days after icv injection of vehicle or AβO. The platform was removed and each animal was allowed a free 60 sec swim. During the probe trial, the time spent in various quadrants of the pool, the number of crossings over the former platform location and the time at first cross (i.e. latency to target) were determined. During the probe trial, no difference in swimming speed was observed between the groups, showing normal physical capability and motivation.

Number of crossings - Mice from the vehicle control group (Group A) exhibited normal behavior with a mean crossing number of 5.3 ± 0.6 (Figure 3). These results are in agreement with previous observations of similar control groups. As expected, a single icv injection of AβO (Group B) resulted in a significant impairment of the cognitive performance when compared to vehicle control mice, with a decreased mean crossing number of 2.2 ± 0.4 (p=0.0010 as compared to control mice).

Mice dosed sub-chronically with donepezil (po) and icv injected with AβO (Group C) exhibited an improved behavior with a mean crossing number of 3.7 ± 0.4. These mice were not different from vehicle control mice (p=0.0502) and significantly different from AβO injected mice (p=0.0214). Similarly, mice dosed acutely (on the test day) with donepezil (ip) and icv injected with AβO (Group L) exhibited an improved behavior with a mean crossing number of 3.8 ± 0.4. These mice were not different from vehicle control mice (p=0.0857) and significantly different from AβO injected mice (p=0.0159).

Mice dosed with Donecopride (9 mg/kg/day) or Flucopride (9 mg/kg/day) in the presence of vehicle (Groups D and E, respectively) exhibited normal cognitive performances with a mean crossing number of 4.6 ± 0.5 and 4.6 ± 0.7. These mice were not different from vehicle control mice (p=0.4003 and p=0.3945 for Donecopride and Flucopride, respectively) and significantly different from AβO injected mice (p=0.0026 and p=0.0077 for Donecopride and Flucopride, respectively).

Mice were dosed with increasing doses of Donecopride and icv injected with AβO (Groups F, G and H). The presence of Donecopride (at all doses) resulted in an inhibition of AβO-induced impairment of long-term memory evaluated using the number of crossings. The maximum effect was reached for a dose of 3 mg/kg/day (Group G). Indeed, these mice exhibited normal behavior with a mean crossing number of 5.1 ± 0.6, not different from vehicle control mice (p=0.6575) and significantly different from AβO injected mice (p=0.0018).

Mice were dosed with increasing doses of Flucopride and icv injected with AβO (Groups I, J and K). The presence of Flucopride (at all doses) resulted in an inhibition of AβO-induced impairment of long-term memory evaluated using the number of crossings. The maximum effect was reached for a dose of 3 mg/kg/day (Group J). Indeed, these mice exhibited normal behavior with a mean crossing number of 4.1 ± 0.6, not different from vehicle control mice (p=0.1668) and significantly different from AβO injected mice (p=0.0242).

Latency to target - Mice from the vehicle control group (Group A) exhibited normal behavior with a mean latency to target of 12.0 ± 1.9 s. These results are in agreement with previous observations of similar control groups. As expected, a single icv injection of AβO (Group B) resulted in a significant impairment of the cognitive performance when compared to vehicle control mice, with an increased mean latency to target of 24.4 ± 4.7 s (p=0.0074 as compared to control mice).

Mice dosed sub-chronically with donepezil (po) and icv injected with AβO (Group C) exhibited an improved behavior with a mean latency to target of 14.6 ± 2.7 s. These mice were not different from vehicle control mice (p=0.6860) and significantly different from AβO injected mice (p=0.0272). Similarly, mice dosed acutely (on the test day) with donepezil (ip) and icv injected with AβO (Group L) exhibited an improved behavior with a mean latency to target of 13.3 ± 2.3 s. These mice were not different from vehicle control mice (p=0.9264) and significantly different from AβO injected mice (p=0.0028).

Mice dosed with Donecopride (9 mg/kg/day) or Flucopride (9 mg/kg/day) in the presence of vehicle (Groups D and E, respectively) were found to exhibit normal cognitive performances with a mean latency to target of 7.2 ± 1.5 s and 12.0 ± 2.1 s. These mice were not different from vehicle control mice (p=0.0524 and p=0.7582 for Donecopride and Flucopride, respectively) and significantly different from AβO injected mice (p=0.0017 and p=0.0082 for Donecopride and Flucopride, respectively).

Mice were dosed with increasing doses of Donecopride and icv injected with AβO (Groups F, G and H). The presence of Donecopride (at all doses) resulted in an inhibition of AβO-induced impairment of long-term memory evaluated using the latency to target. The maximum effect was reached for a dose of 1 mg/kg/day (Group F). Indeed, these mice exhibited normal behavior with a mean latency to target of 9.3 ± 1.4 s, not different from vehicle control mice (p=0.2548) and significantly different from AβO injected mice (p=0.0005).

Mice were dosed with increasing doses of Flucopride and icv injected with AβO (Groups I, J and K). The presence of Flucopride (only at a dose of 9 mg/kg) resulted in an inhibition of AβO-induced impairment of long-term memory evaluated using the latency to target. Indeed, mice from Group K exhibited normal behavior with a mean latency to target of 10.7 ± 1.9 s, not different from vehicle control mice (p=0.6891) and significantly different from AβO injected mice (p=0.0031).

Time in quadrant - Mice from the vehicle control group (Group A) spent significantly more time in the target quadrant (p<0.0001 as compared to time spent in the opposite quadrant). These results are in agreement with previous observations of similar control groups. As expected, a single icv injection of AβO (Group B) resulted in a significant impairment of the cognitive performance when compared to vehicle control mice, with more time spent in the opposite quadrant than in the target quadrant (p=0.0779 when times spent in target vs opposite quadrant are compared). The subchronic or acute dosing of donepezil (Groups C and L) failed to improve AβO-induced impairment of long-term memory using the time spent in the different quadrants as a read-out. Similarly, Donecopride and Flucopride did not improve quadrant frequentation during the probe test.

### Effects of the compounds of the invention on recognition memory

To determine the effect of test items on recognition memory, a NOR test was performed 16 days after icv injection of either vehicle or AβO. Behavioral trials were performed as described in the Experimental Procedures.

Mice from the vehicle control group (Group A) exhibited normal behavior with a mean discrimination index of 0.35 ± 0.04. These results are in agreement with previous observations of similar control groups. As expected, a single icv injection of AβO (Group B) resulted in a significant impairment (p<0.0001) of the cognitive performance when compared to vehicle control mice; with a mean discrimination index of -0.01 ± 0.03, AβO-injected mice were not able to discriminate between novel and familiar objects.

Mice dosed sub-chronically with Donepezil (po) and icv injected with AβO (Group C) exhibited normal behavior with a mean discrimination index of 0.29 ± 0.09. These mice were not different from vehicle control mice (p=0.7439) and significantly different from AβO injected mice (p=0.0192).

Mice dosed acutely (on the test day) with Donepezil (ip) and icv injected with AβO (Group L) exhibited normal behavior with a mean discrimination index of 0.30 ± 0.07. These mice were not different from vehicle control mice (p=0.9158) and significantly different from AβO injected mice (p=0.0030).

Mice dosed with Donecopride (9 mg/kg/day) or Flucopride (9 mg/kg/day) in the presence of vehicle (Groups D and E, respectively) were found to exhibit normal cognitive performances with a mean discrimination index of 0.29 ± 0.04 and 0.36 ± 0.06. These mice were not different from vehicle control mice (p=0.3071 and p=0.8205 for Donecopride and Flucopride, respectively) and significantly different from AβO injected mice (p=0.0007 and p=0.0003 for Donecopride and Flucopride, respectively).

Mice were dosed with increasing doses of Donecopride and icv injected with AβO (Groups F, G and H). The presence of Donecopride (at all doses) resulted in a dose-dependent inhibition of AβO-induced impairment of recognition memory. The maximum effect was reached for a dose of 9 mg/kg/day (Group H). Indeed, these mice exhibited normal behavior with a mean discrimination index of 0.43 ± 0.06, not different from vehicle control mice (p=0.3440) and significantly different from AβO injected mice (p=0.0003).

Mice were dosed with increasing doses of Flucopride and icv injected with AβO (Groups I, J and K). The presence of Flucopride (at all doses) resulted in a dose-dependent inhibition (bell-shaped curve) of AβO-induced impairment of recognition memory. The maximum effect was reached for a dose of 3 mg/kg/day (Group J). Indeed, these mice exhibited normal behavior with a mean discrimination index of 0.46 ± 0.05, not different from vehicle control mice (p=0.1039) and significantly different from AβO injected mice (p=0.0003).

### Conclusion

Altogether, these data strongly suggest that both Donecopride and Flucopride improved cognitive performances of mice challenged with AβO.

### Example 3

This example shows the anti-amnesic, anti-amyloid and anti-inflammatory effects of Donecopride and Flucopride in chronic administration to the 5XFAD mouse model of Alzheimer's disease.

### Study rationale

The objective of the study was to evaluate the potential anti-amnesic, anti-amyloid and anti-inflammatory effect of Donecopride and Flucopride in chronic administration in a mouse model of Alzheimer's disease.

The mouse model used was the 5XFAD strain (referred at the Jackson Laboratory as "B6SJL-Tg(APPSwFILon,PSEN1*M146L*L286V)6799Vas/Mmjax", MMRRC Stock No: 34840-JAX or "B6.Cg-Tg(APPSwFILon,PSEN1*M146L*L286V)6799Vas/ Mmjax, MMRRC Stock No: 34848-JAX).

These 5XFAD transgenic mice overexpress mutant human APP(695) with the Swedish (K670N, M671L), Florida (I716V), and London (V717I) Familial Alzheimer's Disease (FAD) mutations along with human PS1 harboring two FAD mutations, M146L and L286V. Both transgenes are regulated by the mouse Thy1 promoter to drive overexpression in the brain. 5XFAD mice recapitulate major features of Alzheimer's Disease amyloid pathology and are a useful model of intraneuronal Aβ42 induced neurodegeneration and amyloid plaque formation.

### Methods and Experimental Design

### Mice

Animal experiments were carried out in accordance with the Directive by the Council of the European Communities of November 24, 1986 (86/609/EEC). All efforts were made to minimize animal suffering and to reduce the number of mice used. The generation of 5XFAD mice was described in Oakley et al. (2006) J. Neurosci. 26:10129-10140. These transgenic mice overexpress both human APP (695) harboring the Swedish (K670N, M671L), Florida (I716V) and London (V717I) familial AD (FAD) mutations and human Presenilin 1 (PS1) harboring the two FAD mutations M146L and L286V. Expression of both transgenes is regulated by neuronal-specific elements of the mouse *Thy1* promoter. The 5XFAD strain (B6/SJL genetic background) was maintained by crossing hemizygous transgenic mice with B6/SJL F1 breeders. The 5XFAD strain (C57BL/6J genetic background) was maintained by crossing hemizygous transgenic mice with C57BL/6J F1 breeders. All animals were genotyped by PCR using tail genomic DNA. Transgenic and WT mice were bred in our animal facility, had access to food and water ad *libitum* and were housed under a 12 h light-dark cycle at 22-24°C. Female 5XFAD mice were used in this study. For each experiment, the age of mice is indicated in months (with a two-week range).

### Drugs

Donecopride (Donecopride fumarate, MR 31155) and Flucopride (Flucopride fumarate, MR 31193) were synthesized in the CERMN, Caen, France.

RS 67333 (1-(4-amino-5-chloro-2-methoxy-phenyl)-3-(1-butyl-4-piperidinyl)-1-propanone), was used as a reference 5-HT₄ receptor agonist and was purchased from Tocris Bioscience (R&D Systems Europe, Lille, France).

Compounds were resuspended in DMSO (37.5 µg/µl, stored at -20°C) and freshly diluted 1:250 in 0.9% NaCl prior administration. Vehicle solution (0.9% NaCl, 0.4% DMSO) was used as control.

### Animal treatments

5XFAD mice received chronic treatments with drugs or vehicle according to 2 different protocols. In each experiment, drugs or vehicle solution were administered i.p. twice a week (1 mg/kg). In "Protocol 1", mice (n = 6-7 mice per group) were treated with compounds for two months, from 2 to 4 months of age. In "Protocol 2", mice (n = 7) were treated with compounds for three months, from 1 month to 4 months of age. At the end of each protocol, mice were anesthetized with a mixture of 100 mg/kg ketamine and 10 mg/kg xylazine in saline solution and perfused transcardially with PBS. Brains were quickly isolated on ice, the olfactory bulbs and cerebellum removed and the two hemispheres divided. One hemisphere was frozen on dry ice and stored at -80°C for biochemical analysis, while the other was post-fixed in 4 % PFA for immunohistochemistry (IHC).

### Cerebrospinal fluid (CSF) collection

Mice were anesthetized and mounted onto a stereotaxic instrument. The neck skin was cut and subcutaneous tissue and muscles separated with the help of micro-retractors (Fine Science Tools, Heidelberg, Germany). Mice were then laid down so that the head formed an angle of about 135° with the body (Liu and Duff (2008) J. Vis. Exp. 10:960). A capillary tube (Borosilicate glass, B100-75-10, Sutter Instruments, Novato, California, USA) was used to punch the *dura mater* of the *cisterna magna.* CSF was collected by capillary action and transferred to 0.5 mL microtubes, immediately frozen on dry ice and stored at -80°C until use. Once thawed, samples were heated at 60°C for 5 min as described in the international application WO 2008/009868 and analyzed without further freezing-thawing cycles.

### Brain extract preparation

Brain hemispheres, frontal cortex and hippocampus of 5XFAD mice and controls were thawed, weighed and homogenized in 4 volumes of tris-saline (50 mM Tris-HCI pH = 7.4, 150 mM NaCl) with a protease inhibitor cocktail (Roche Applied Science, Meylan, France). The resulting homogenates were centrifuged at 54,000 x g for 20 minutes and supernatants (the "soluble fraction") collected and aliquoted for storage at -80°C. Pellets were resuspended by brief sonication in 10 volumes of 6 M guanidine HCI in 50 mM Tris-HCI, pH = 7.6 and centrifuged again at 26,500 x g for 20 minutes. Supernatants (the "insoluble fraction") were aliquoted and stored at -80°C (Morishima-Kawashima et al. (2000) Am. J. Pathol. 157:2093-2099).

### Quantification of Aβ₄₀ and Aβ₄₂

ELISA kits from IBL International (Hamburg, Germany) for the dosage of Aβ₄₀ (human amyloid β (1-40) assay kit, #27713) or Aβ₄₂ (human amyloid β (1-42) assay kit, #27719) were used according to the manufacturer's instructions. Reactions were read at 620 nm and 450 nm using an Infinite 2000 luminescence counter. The obtained values were normalized to the protein concentration of each sample, measured using a BCA protein assay (Sigma-Aldrich).

### Immunohistochemistry

Thirty-micrometer-thick sections were cut using a vibratome (Microm HM 650V, Thermo Scientific, Saint Herblain, France) and stored in cryoprotectant medium at -20°C. For the labeling of amyloid plaques, free-floating tissue sections of frontal cortex, hippocampus and entorhinal cortex (coordinates from the bregma: frontal cortex = 1.98 mm, hippocampus = -1.94 mm, entorhinal cortex = -3.08 mm) were extensively washed in PBS and then incubated in blocking solution (PBS; 3% BSA; 0.1% Triton X-100) for 1 h. Sections were stained with Hoechst dye (1:1000, Life Technologies, Saint Aubin, France) for 15 minutes to detect cell nuclei and then with freshly prepared thioflavin T solution (#T3516-5G, Sigma-Aldrich) (final concentration: 0.01 mg/ml in blocking buffer) for 15 minutes. After washing in 70 % ethanol for 5 minutes, samples were mounted on poly-lysine slides with coverslips. For GFAP (glial fibrillary acidic protein) staining, free-floating brain sections were blocked as before and incubated with polyclonal rabbit anti-GFAP (1:1000, Z0334, Dako, Les Ullis, France) at 4°C overnight. After thioflavin T staining and washing with 70 % ethanol, the secondary Alexafluor 594 goat anti-rabbit antibody (1:1000, A11012, Life Technologies) was added for 2 h. PBS washes and mounting were performed as described before.

### Image acquisition and analysis

Images were acquired with an Axiolmager Z1 microscope (Carl Zeiss S.A.S., Marly le Roi, France). Analysis of thioflavin T staining was performed blindly and data are presented as the mean number of particles per mm² in two to three tissue sections from the same brain area/animal (Image J software). GFAP expression was quantified using Image J software and results are expressed as area fractions.

### Novel object recognition test

The cognitive performance of mice was tested using the novel object recognition (NOR) test (Bevins and Besheer (2006) Nat. Protoc. 1:1306-1311). Animals were extensively handled during drug treatment prior to the test onset. Each day, mice were allowed to familiarize with the test room for at least 1 h prior to the test. Testing was carried out in a Plexiglas box (width: 35 cm, length: 20 cm, height: 20 cm) placed in a dimly lit room. On day 1 and 2, each mouse was habituated to the empty box for 10 min/day. On day 3, two objects (constructed out of plastic toys) were positioned in the cage, 5 cm away from the opposing walls. During the training session, each animal was placed between the two objects, facing the wall and then was allowed to explore the objects for 5 min. Mice were then returned to their home cage and 24 h later went through a 5 min test session in which one of the two (familiar) objects was replaced by a new one (novel). The whole experiment was video-recorded and object exploration [time spent by the mouse nose in contact with the object or by sniffing it at a distance ≤ 1 cm] was blindly measured. Two parameters were considered: 1) the exploration time (sec) spent by the animal interacting with the two familiar objects during the training session and 2) the exploration time spent by the animal interacting with the novel object relative to the total exploration time ([novel/(familiar + novel)] × 100) during the test. A discrimination index was also calculated ([novel - familiar]/[familiar + novel]).

### Statistical analysis

All values are expressed as the mean ± SEM. Significant effects of treatments were determined by ANOVA analysis followed by Tukey's *post hoc* tests in the case of multiple comparison groups. In all other cases, the unpaired Student's *t* test was used. For all statistical tests, *P* < 0.05 was considered significant. Analysis was performed with GraphPad Prism 7 (GraphPad Software, La Jolla, CA).

### Results

### Effects of Donecopride and Flucopride on amyloid load in 5XFAD brains

27 female 5XFAD mice (B6/SJL genetic background) were used in this study. Mice received vehicle or compounds (1 mg / kg) i.p., twice a week for two months, from 2 to 4 months of age.

Aβ₄₀ and Aβ₄₂ were quantified from brain extract in soluble and insoluble fractions. Results are expressed in % of vehicle.

The results obtained are presented in table 6 below:

**Table 6: Aβ₄₀ and Aβ₄₂ levels (% of controls)**

| | **Soluble fraction** | | | **Insoluble** fraction | | |
|---|---|---|---|---|---|---|
| | **A**β₄₀ | **SEM** | **n** | **A**β₄₀ | **SEM** | **n** |
| **Vehicle** | 100.00 | 11.38 | 7 | 100.00 | 14.56 | 7 |
| **Donecopride** | 110.18 | 10.68 | 7 | 79.97 | 6.40 | 7 |
| **Flucopride** | 110.51 | 16.67 | 7 | 97.16 | 10.43 | 7 |
| | | | | | | |

| | **A**β₄₂ | **SEM** | **n** | **Aβ**₄₂ | **SEM** | **n** |
|---|---|---|---|---|---|---|
| **Vehicle** | 100.00 | 11.26 | 6 | 100.00 | 6.70 | 7 |
| **Donecopride** | 65.93* | 8.36 | 7 | 67.85* | 12.59 | 6 |
| **Flucopride** | 30.19**** | 8.04 | 7 | 73.16 | 4.62 | 7 |

| | | | | | | |
|---|---|---|---|---|---|---|
| *: p < 0.05 versus vehicle, determined by one-way ANOVA followed by a Tukey's test. | | | | | | |

The values of the vehicle groups presented in Table 6 were as follows:
∘ soluble faction:
   ▪ Aβ₄₀ = 9.48 ± 1.08 ng/mg of protein,
   ▪ Ap₄₂ = 681.01 ± 99.13 ng/mg of protein;
∘ insoluble fraction:
   ▪ Aβ₄₀ = 0.24 ± 0.02 ng/mg of protein,
   ▪ Ap₄₂ = 10,834.16 ± 726.36 ng/mg of protein.

**Anti-amyloid effect.** In the soluble fraction of 5XFAD mouse brains, the chronic administration of Donecopride and Flucopride (1 mg/kg, twice a week) was able to significantly reduce Aβ₄₂ levels (*p < 0.05 versus vehicle for Donecopride and ****p < 0.0001 versus vehicle for Flucopride, determined by one-way ANOVA followed by Tukey's test) without any change on Aβ₄₀ levels. Similarly, in the insoluble fraction, donecopride reduced Aβ₄₂ load in the mouse brains (*p < 0.05 versus vehicle, determined by one-way ANOVA followed by Tukey's test). Insoluble Aβ₄₀ levels were unaffected by both drugs.

### Effects of Donecopride and Flucopride on memory, amyloid load in CSF, amyloid plaques and astrogliosis in 5XFAD mice

28 female 5XFAD mice (C57BL/6J genetic background) were used in this study. Mice received vehicle or compounds (1 mg / kg) i.p., twice a week for three months, from 1 to 4 months of age.

At the end of the treatment, mice were evaluated for long-term memory performance in novel object recognition test (NOR). The inter-session interval was 24 hours. As no mice failed to explore either of the two objects or explored both objects for less than 10 sec during the training session, all the mice were included in the data analysis.

**Anti-amnesic effect.** One-month-old 5XFAD females and WT littermates were treated with RS 67333, vehicle, Donecopride or Flucopride (1 mg/kg, twice a week for 3 months) and then recognition memory was assessed with the novel object recognition (NOR) test. Habituation sessions were started 3 days after the end of treatment to avoid interference with acute effects. The interval between training session and test was of 24h. Vehicle-treated mice presented impaired memory performance and were not able to discriminate the novel object versus the familiar one, as shown by similar exploration time and non-significant discrimination index. RS 67333-treatment reverses cognitive impairment as indicated by the higher percentage of time devoted to the exploration of the novel object in comparison to mice treated with vehicle (**p < 0.01 versus familiar object, determined by two-way ANOVA followed by a Sidak's test). Similarly, Donecopride and Flucopride chronic treatments were able to prevent memory alteration (**p < 0.01 versus familiar object for Donecopride, *p < 0.05 versus familiar object for Flucopride, determined by two-way ANOVA followed by a Sidak's test). Discrimination index of the 3 compound-treated groups were different from zero, the chance level (determined by unpaired Student's t test). The total exploration time during the training session did not differ significantly in the four groups, indicating that the recognition memory improvement in 5XFAD mice treated with Donecopride or Flucopride is not secondary to an increase of the exploratory activity.

**Amyloid load in CSF.** Aβ₄₂ concentration in cerebrospinal fluid (CSF) of 5XFAD mice treated with RS 67333, Donecopride or Flucopride (1 mg/kg, twice a week for 3 months) was not significantly different compared to controls, indicating that chronic 5-HT₄ receptor stimulation did not affect amyloid levels in CSF (determined by one-way ANOVA followed by a Tukey's test).

**Anti-amyloid effect.** Frontal cortex, hippocampus and entorhinal cortex were analyzed as representative areas of 5XFAD mouse brain (frontal, median and caudal sections) and of human brain regions that are affected early by Alzheimer's disease and are highly enriched in amyloid deposits.

Donecopride treatment (1 mg/kg, twice a week for 3 months) significantly decreased the number of amyloid plaques compared to vehicle in frontal cortex (reduction of 16 ± 3%, **p < 0.01 compared with vehicle) and in entorhinal cortex (reduction of 24 ± 4%, **p < 0.01 compared with vehicle). A significant reduction of the plaque number was also observed in entorhinal cortex after Flucopride treatment (1 mg/kg, twice a week for 3 months) with a reduction of 28 ± 6%, relative to control (**p < 0.01 compared with vehicle). No significant effect was seen following RS 67333-treatment (1 mg/kg, twice a week for 3 months) neither in frontal cortex nor in entorhinal cortex. Amyloid plaque number in hippocampus of 5XFAD mice treated with RS 67333, Donecopride or Flucopride (1 mg/kg, twice a week for 3 months) was not significantly different compared to controls. Statistics were determined by one-way ANOVA followed by a Tukey's test.

**Anti-inflammatory effect.** Immunohistochemical assessment of astroglial (anti-GFAP antibody, Glial fibrillary acidic protein) showed that Donecopride and RS 67333 chronic treatments (1 mg/kg, twice a week for 3 months) significantly reduced astrogliosis (54 ± 10 % and 62 ± 6 % decrease of GFAP staining in entorhinal brain slices of treated animals in comparison to controls that received vehicle, respectively, *p < 0.01 compared with vehicle). In entorhinal cortex, Flucopride treatment (1 mg/kg, twice a week for 3 months) induced a reduction of 51 ± 5 % of GFAP staining that was close to significance (p = 0.06). However, GFAP staining in frontal cortex or hippocampus of 5XFAD mice treated with RS 67333, Donecopride or Flucopride (1 mg/kg, twice a week for 3 months) was not significantly different compared to controls. Statistics were determined by one-way ANOVA followed by a Tukey's test.

### Conclusion

The study demonstrates that Donecopride and Flucopride exert anti-amyloid effects (reduction of Aβ₄₂ levels and amyloid plaque) and anti-inflammatory effects (reduction of astrogliosis) upon chronic administration to 5XFAD mice, a transgenic mouse model of Alzheimer's disease. These effects are mostly prominent in entorhinal cortex and frontal cortex. Moreover, chronic administration of Donecopride and Flucopride is able to exert an anti-amnesic effect and to prevent the appearance of memory deficits in 5XFAD mice.

All these effects are comparable, and sometimes greater, to those of RS 67333 (5-HT₄ receptor agonist chosen as reference), pointing out Donecopride and Flucopride as promising drug against Alzheimer's disease.

## Claims

1. Compound of the following formula (I): 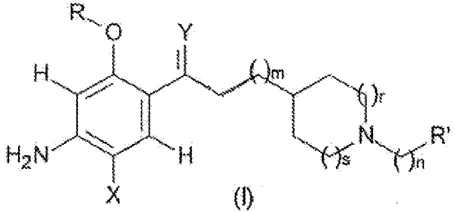 wherein:
X represents
a hydrogen atom, or
a halogen atom (Hal), where (Hal) is fluorine, chlorine, bromine or iodine, or
a straight- or branched-chain Cₚ(Hal)₂ₚ₊₁ polyhalogenoalkyl group, where p=1, 2, 3 or 4, (Hal) having the same meaning as indicated above;
Y represents
an oxygen atom, or
a sulfur atom, or
an N-R" radical where R" represents a hydrogen atom, an -OH radical or a straight- or branched-chain C_{q}H_{2q+1} alkyl radical, where q=1, 2, 3 or 4;
m is an integer selected from 1, 2 and 3;
n is an integer selected from 0, 1, 2 and 3;
r and s are integers whose values are: r=s=0; or r=s=1; or r=s=2; or r=0 and
s=1; or lastly r=0 and s=2;
R represents
a hydrogen atom, or
a straight- or branched-chain C₁-C₅ alkyl group capable of carrying one or more F atoms;
R' represents
a branched-chain C₁-C₆ alkyl radical, or
a C₃-C₁₀ cycloalkyl or C₅-C₁₃ bicyclic group, capable of carrying one or more R groups and of possessing an oxygen atom, or a nitrogen atom that can be substituted by R, or a sulfur atom or a radical -SO₂₋ or -SO-,
as well as its enantiomers or diastereoisomers and its racemics, its acid salts, its hydrates or its solvation products,
for use as neuroprotective agent in the prevention and/or treatment of a neurodegenerative disease.

2. Compound for its use according to claim 1, wherein X in the formula (I) represents a halogen atom.

3. Compound for its use according to claim 1 or 2, wherein Y in the formula (I) represents an oxygen atom.

4. Compound for its use according to any one of claims 1 to 3, wherein all of m, n, r and s in the formula (I) have the value 1.

5. Compound for its use according to any one of claims 1 to 4, wherein R in the formula (I) represents H, CH₃, CH₂CH₃ or CH₂-CH₂F.

6. Compound for its use according to any one of claims 1 to 5, wherein R' in the formula (I) represents a radical selected from the group consisting in the radicals cyclopropyl, cyclobutyl, cyclopentyl, cyclohexyl, and 4-piperidine.

7. Compound for its use according to claims 2 to 6, wherein R in the formula (I) represents a methyl radical and R' a C₄-C₇ cycloalkyl radical.

8. Compound for its use according to any one of claims 1 to 7, wherein the compound is the Donecopride compound of the following formula (II):

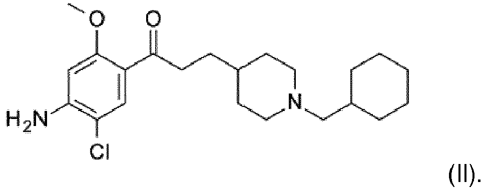

9. Compound for its use according to any one of claims 1 to 7, wherein the compound is the Flucopride compound of the following formula (III):

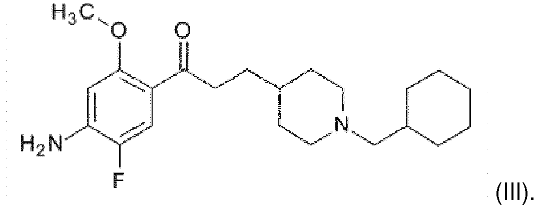

10. Compound for its use according to any one of claims 1 to 10, wherein the neurogenerative disease is selected from the group consisting in Alzheimer's disease, Parkinson's disease, Huntington's disease, multiple system atrophy, motor neurone diseases including amyotrophic lateral sclerosis, Down syndrome and frontotemporal degenerations.
